# EUROPEAN PATENT SPECIFICATION

(11) **EP 2 018 359 B1**
(45) Date of publication and mention of the grant of the patent: **17.08.2011**
(21) Application number: 07729195.3
(22) Date of filing: 16.05.2007
(51) Int. Cl.: C07C 1/20

(54) **PROCESS FOR THE PREPARATION OF AN OLEFIN**
VERFAHREN ZUR HERSTELLUNG EINES OLEFINS
PROCÉDÉ DE PRÉPARATION D'UNE OLÉFINE

(30) Priority: 19.05.2006 EP 06114277
(43) Date of publication of application: 28.01.2009
(73) Proprietor: Shell Internationale Research Maatschappij B.V., 2596 HR The Hague (NL)
(72) Inventor: CHEWTER, Leslie Andrew, 1031 CM Amsterdam (NL); VERHAAK, Michiel Johannes Franciscus Maria, 1031 CM Amsterdam (NL); VAN WESTRENEN, Jeroen, 1031 CM Amsterdam (NL)
(74) Representative: Matthezing, Robert Maarten
(86) International application number: PCT/EP2007/054747
(87) International publication number: WO 2007/135049

(56) References cited:
- WO-A-02/10098
- US-A1- 2003 078 463
- SVELLE ET AL: "Conversion of methanol into hydrocarbons over zeolite H-ZSM-5: ethene formation is mechanistically separated from the formation of higher alkenes", JOURNAL OF THE AMERICAN CHEMICAL SOCIETY., vol. 128, no. 46, 26 October 2006 (2006-10-26), pages 14770-14771, XXXX ISSN: 0002-7863

## Description

### Field of the invention

This invention relates to a process for the preparation of an olefin, such as ethene and/or propene. In specific this invention relates to a process for the conversion of oxygenates into olefins.

### Background of the invention

Processes for the preparation of olefins are known in the art, such as for example described in WO02/10098.

US2003/0078463 describes a process for making ethylene and propylene from an oxygenate feed using two or more zeolite catalysts.

One of the methods described comprises contacting in a first stage an oxygenate with a first zeolite catalyst to form an olefin product; separating a butylene containing stream from the olefin product; and contacting in a second phase the butylene containing stream with a second zeolite catalyst to form a second olefin product.

In passing, it is described that hydrocarbons can also be included as part of the feedstock in the first stage, i.e. as co-feed. It is mentioned that such hydrocarbon co-feed can include olefins, paraffins, alkylaromatics, aromatics or mixtures thereof. Preferred co-feeds are C₄+ hydrocarbon mixtures which are obtained from separation and recycle of olefin product. An embodiment is described wherein the product stream of the second stage is separated into an ethylene stream, a propylene stream and a C₄+ stream. It is stated that a portion of the later stream may be recycled to the oxygenate feed.

In the only example, pure methanol is converted by a two-step process into several olefins.

Buying in of external hydrocarbon co-feed could be economically disadvantageous.

It would be desirable to have an improved process, wherein an olefinic co-feed is used which is generated in an economically attractive manner.

### Summary of the invention

It has now been found that the process can be made more economical by starting up the process with a co-feed of olefin from an external source and continuing the process with a co-feed of olefin prepared by the process itself.

Accordingly the present invention provides a process for the preparation of an olefin comprising
reacting an oxygenate feed and an olefinic co-feed in a reactor in the presence of a zeolite to obtain a reaction product containing one or more olefins;
wherein the olefinic co-feed is at least partially obtained ex-situ during a first phase and at least partially obtained in-situ during a subsequent second phase.

As a result an economical process is obtained wherein an olefinic co-feed is generated in a economically advantageous manner. Expensive olefinic co-feed from an external source is only needed for a limited amount of time and an economically more attractive process is obtained.

### Detailed description of the invention

By an olefinic co-feed is understood a feed containing one or more olefins. The olefinic co-feed can contain one olefin or a mixture of olefins. Preferably the olefinic co-feed contains a mixture of olefins. Apart from olefins, the olefinic co-feed may contain other hydrocarbon compounds, such as for example paraffinic, alkylaromatic, aromatic compounds or mixtures thereof. Preferably the olefinic co-feed comprises more than 50 wt%, more preferably more than 80 wt%, still more preferably more than 90 wt% and most preferably in the range from 95 to 100 wt% of olefin(s), based on the total weight of olefinic co-feed. An especially preferred olefinic co-feed consists essentially of olefin(s).

Any non-olefinic compounds in the olefinic co-feed are preferably paraffinic compounds. If the olefinic co-feed contains any non-olefinic hydrocarbon, these are preferably paraffinic compounds. Such paraffinic compounds are preferably present in an amount of less than 10 wt%, more preferably in an amount in the range from 0 to 5 wt%, still more preferably in the range from 0 to 1 wt% and most preferably in an amount of less than 0.5 wt%, based on the total weight of olefinic co-feed.

By an olefin is understood an organic compound containing at least two carbon atoms connected by a double bond. A wide range of olefins can be used. The olefin can be a mono-olefin, having one double bond, or a poly-olefin, having two or more double bonds. Preferably olefins present in the olefinic co-feed are mono-olefins.

The olefin(s) can be a linear, branched or cyclic. Preferably olefins present in the olefinic co-feed are linear or branched olefins.

Preferred olefins have in the range from 2 to 12, preferably in the range from 3 to 10, and more preferably in the range from 4 to 8 carbon atoms.

Examples of suitable olefins that may be contained in the olefinic co-feed include ethene, propene, 1-butene, 2-butene, iso-butene (2-methyl-1-propene), 1-pentene, 2-pentene, 2-methyl-1-butene, 2-methyl-2-butene, 3-methyl-1-butene, 3-methyl-2-butene, 1-hexene, 2-hexene, 3-hexene, 2-methyl-1-pentene, 2-methyl-2-pentene, 3-methyl-1-pentene, 3-methyl-2-pentene, 4-methyl-1-pentene, 4-methyl-2-pentene, 2,3-dimethyl-1-butene, 2, 3-dimethyl-2-butene, 3,3-dimethyl-1-butene, cyclopentene, methylcyclopentene or cyclohexene, heptenes, octenes, nonenes and decenes. The preference for specific olefins in the olefinic co-feed may depend on the purpose of the process.

In one embodiment, where the purpose of the process is to prepare ethene and/or propene, the olefinic co-feed preferably contains only olefins having 4 or more carbon atoms (i.e. C₄+ olefins), such as butenes, pentenes, hexenes and heptenes. More preferably, in such case, the olefins are essentially butenes, pentenes and hexenes. In such a case it may be preferred to use an olefinic co-feed which consists of hydrocarbons containing more than 4 carbon atoms, also referred to as a C₄+ hydrocarbon feed.

In another embodiment, where the purpose of the process is to prepare pentenes and/or hexenes, the olefinic co-feed preferably contains olefins having 4 or less carbon atoms (i.e. C₄- olefins), such as butenes, propene and ethene. In such a case it may be preferred to use an olefinic co-feed which consists of hydrocarbons containing less than 4 carbon atoms, also referred to as a C₄- hydrocarbon feed.

In a still further embodiment, where the purpose of the process is to prepare ethene, propene, pentene and/or hexene, the olefinic co-feed preferably contains only olefins having 4 carbon atoms. In such a case it may be preferred to use an olefinic co-feed which consists of hydrocarbons containing 4 carbon atoms, also referred to as a C₄ hydrocarbon feed.

During the first phase of the process, the olefinic co-feed is at least partially obtained ex-situ.

By "at least partially obtained ex-situ" is understood that at least part of the olefins in the olefinic co-feed are not prepared by the process itself. Such olefins may for example be obtained from a steam cracker, a catalytic cracker, alkane dehydrogenation (e.g. propane or butane dehydrogenation). Further, such olefins can be bought from the market.

In a special embodiment the olefins for such start-up are obtained from a previous process that converted oxygenates, with or without olefinic co-feed, to olefins. Such a previous process may have been located at a different location or it may have been carried out at an earlier point in time. The olefins may have been stored or shipped.

During the subsequent second phase, the olefinic co-feed is at least partially obtained in-situ.

By "at least partially obtained in-situ" is understood that at least part of the olefins in the olefinic co-feed are directly or indirectly prepared by the process according to the invention. The olefins can for example be obtained directly by recycling part of the olefins in the reaction product of the process; or indirectly by converting part of the reaction product of the process, containing one or more olefins, into a further product containing olefins and recycling part of such further product.

An example of an olefinic co-feed that is indirectly prepared from the process according to the invention is an olefinic co-feed that contains lower olefins, such as ethene, propene and/or butenes, which are obtained by cracking higher olefins such as, heptenes and/or octenes, which are in turn derived from the reaction product of the process.

Another example of an olefinic co-feed that is indirectly prepared from the process according to the invention is an olefinic co-feed that contains higher olefins such as butenes, pentenes and/or hexenes, which higher olefins are obtained by oligomerization of lower olefins, such as ethene or propene, which are in turn derived from the reaction product of the process.

An olefinic co-feed is used in the second phase comprising olefins which are (directly) generated in the process of the invention. Such olefins can be separated from other olefins in the reaction product and recycled. Hence, the process of the invention comprises the steps of
a) reacting an oxygenate feed and an olefinic co-feed in a reactor in the presence of a zeolite to obtain a reaction product containing one or more olefins;
b) separating the reaction product obtained in step a) in at least a product fraction containing olefins and a further fraction containing olefins;
c) recycling at least part of the further fraction containing olefins to step a);
wherein the olefinic co-feed in step a) is at least partially obtained ex-situ during a first phase and wherein the olefinic co-feed in step a) consists essentially of recycled olefins from step c) during a subsequent second phase.

Preferably the part of the olefinic co-feed obtained ex-situ gradually decreases in time and the part of the olefinic co-feed obtained in-situ gradually increases in time.

During the first phase, also referred to as start-up phase or initiating step, preferably in the range from 20 to 100 wt% of the olefinic co-feed is obtained ex-situ and in the range from 0 to 80 wt% of the olefinic co-feed is obtained in-situ. During the subsequently second phase, also referred to as continuation phase or propagating step, preferably in the range from 80 to 100 wt% of the olefinic co-feed is obtained in-situ and in the range from 0 to 20 wt% of the olefinic co-feed is obtained ex-situ. More preferably the amount of olefinic co-feed obtained in-situ in the subsequent second phase lies in the range from 90 to 100 wt% and still more preferably in the range from 98 to 100 wt%, based on the total weight of olefinic co-feed.

Most preferably the olefinic co-feed during the second phase is obtained only in-situ. In this case the first phase may be defined as the phase in which still part of the olefinic co-feed is obtained ex-situ.

The first phase can range from 0.01 seconds to 30 days. More preferably the first phase takes from 1 minute to 2 days and still more preferably the first phase takes from 0.5 to 16 hours.

By an oxygenate is understood a compound comprising at least one oxygen-bonded alkyl group. The oxygen-bonded alkyl group preferably comprises 1 to 4 carbon atoms, more preferably 1 or 2 carbon atoms and most preferably 1 carbon atom. The oxygenate can comprise one or more of such oxygen-bonded C₁-C₄ alkyl groups. Preferably, however, the oxygenate comprises one or two oxygen-bonded C₁-C₄ alkyl groups. Examples of preferred oxygenates include alcohols, such as methanol, ethanol, isopropanol, ethylene glycol, propylene glycol; and ethers, such as dimethylether, diethylether, methylethylether, tetrahydrofuran and dioxane.

Preferably the oxygenate is chosen from the group of dimethylether, diethylether, methylethylether, methanol, ethanol and isopropanol.

More preferably an oxygenate is used having at least one oxygen-bonded C₁ or C₂ alkyl group, still more preferably at least one oxygen-bonded C₁ group. Most preferably the oxygenate is methanol or dimethylether. In a preferred embodiment, where the oxygenate is methanol, such methanol is obtained from natural gas. For example by a process as described in Industrial Organic Chemistry 3rd edition page 28.

In another preferred embodiment the oxygenate is obtained through fermentation of biomaterials. For example by a process as described DE-A-10043644.

The preferred molar ratio of oxygenate to olefin in the olefinic co-feed depends on the specific oxygenate used and the number of reactive oxygen-bonded alkyl groups therein. The ratio of mol oxygenate to mol olefin lies in the range of 10:1 to 1:10, more preferably in the range of 5:1 to 1:5.

In a preferred embodiment wherein the oxygenate comprises only one oxygen-bonded alkyl group, such as for example methanol or ethanol, the molar ratio preferably lies in the range from 5:1 to 1:5.

In another preferred embodiment wherein the oxygenate comprises two oxygen-bonded alkyl group, such as for example dimethylether, the molar ratio preferably lies in the range from 5:2 to 1:10.

The process is carried out in presence of zeolite. By a zeolite is understood a crystalline silica-alumina compound.

This zeolite is a one-dimensional zeolite having 10-membered ring channels.

These are understood to be zeolites having only 10-membered ring channels in one direction which are not intersected by other 8, 10 or 12-membered ring channels from another direction.

Preferably, the zeolite is selected from the group of TON-type (for example ZSM-22), MTT-type (for example ZSM-23), STF-type (for example SSZ-35), SFF-type (for example SSZ-44) and EU-2-type/ ZSM-48 zeolites.

MTT-type catalysts are more particularly described in e.g. US-A-4,076,842. For purposes of the present invention, MTT is considered to include its isotypes, e.g., ZSM-23, EU-13, ISI-4 and KZ-1.

TON-type zeolites are more particularly described in e.g. US-A-4,556,477. For purposes of the present invention, TON is considered to include its isotypes, e.g., ZSM-22, Theta-1, ISI-1, KZ-2 and NU-10.

EU-2-type zeolites are more particularly described in e.g. US-A-4,397,827. For purposes of the present invention, EU-2 is considered to include its isotypes, e.g., ZSM-48.

In a further preferred embodiment a zeolite of the MTT-type, such as ZSM-23, or a TON-type, such as ZSM-22 is used. Of these a zeolite of the MTT-type is especially preferred. When using a zeolite of the MTT-type or TON-type the process according to the invention is especially advantageous. By ensuring the presence of an olefinic co-feed during all phases of the process, for example both during a start-up phase or initiating step and during a continuation phase or propagating step, the functioning of the MTT-type or TON-type zeolite can be improved. The presence of an ex-situ obtained olefinic co-feed during a first phase (e.g. a start-up phase or initiating step), where there is no recycle product available yet, leads to an advantageous good conversion at the start-up of the process. After sufficient olefinic product has been formed, part of such olefinic product can be recycled as in-situ olefinic co-feed to the process.

In case a MFI-type zeolite (e.g. ZSM-5) is used as a catalyst the process of the invention is also especially advantageous. By ensuring the presence of an olefinic co-feed during all phases of the process, both during a start-up phase or initiating step and during a continuation phase or propagating step, the functioning of the MFI-type zeolite can be improved. The presence of an ex-situ obtained olefinic co-feed during a first phase (e.g. a start-up phase or initiating step), where there is no recycle product available yet, leads to a reduced catalyst degradation and a reduced methane make.

Preferably a zeolite in the hydrogen form is used, e.g., HZSM-22, HZSM-23, H-ZSM-35 and HZSM-48. Preferably at least 50% w/w, more preferably at least 90% w/w, still more preferably at least 95% w/w and most preferably 100% of the total amount of zeolite used is zeolite in the hydrogen form. When the zeolites are prepared in the presence of organic cations the zeolite may be activated by heating in an inert or oxidative atmosphere to remove the organic cations, for example, by heating at a temperature over 500 °C for 1 hour or more. The hydrogen form can then be obtained by an ion exchange procedure with ammonium salts followed by another heat treatment, for example in an inert or oxidative atmosphere at a temperature over 500 °C for 1 hour or more. The latter zeolites are also referred to as being in the ammonium form.

Preferably the zeolite has a silica to alumina ratio (SAR) in the range from 1 to 500. Preferably the zeolite has a SAR in the range from 10 to 200.

The zeolite can be used as such or in combination with a so-called binder material. When used in the reaction, the zeolite as such or the zeolite in combination with a binder material, are hereafter also referred to as zeolite catalyst.

It is desirable to provide a catalyst having good crush strength, because in an industrial environment the catalyst is often subjected to rough handling, which tends to break down the catalyst into powder-like material. The later causes problems in the processing. Preferably the zeolite is therefore incorporated in a binder material. Examples of suitable binder materials include active and inactive materials and synthetic or naturally occurring zeolites as well as inorganic materials such as clays, silica, alumina, silica alumina and aluminosilicate. For present purposes, inactive materials of a low acidity, such as silica, are preferred because they may prevent unwanted side reactions which may take place in case a more acidic material, such as alumina is used. Preferably the catalyst used in the process of the present invention comprises, in addition to the zeolite, 2 to 90 wt%, preferably 10 to 85 wt% of a binder material.

The process of the present invention can be carried out in a batch, continuous, semi-batch or semi-continuous manner. Preferably the process of the present invention is carried out in a continuous manner.

The reactor used in the process of the invention may be any reactor known to the skilled person and may for example contain a fixed bed, moving bed, fluidized bed and the like.

Conventional catalyst regeneration techniques can be employed. The zeolite used in the process of the present invention can have any shape known to the skilled person to be suitable for this purpose, for it can be present in the form of tablets, rings, extrudates, etc. extruded catalysts can be applied in various shapes, such as, cylinders and trilobes. If desired, spent zeolite catalyst can be regenerated and recycled to the process of the invention.

The process can be carried out over a wide range of temperatures and pressures. Suitably, however, the hydrocarbon feed is contacted with the zeolite at a temperature in the range from 200 °C to 550 °C, preferably from 200 °C to 500 °C, more preferably in the range from 250 °C to 450 °C and at an absolute pressure in the range from 1 to 5 bar, more preferably in the range from 1 to 3 bar.

Preferably the oxygenate feed and olefinic co-feed are fed to the process according to the invention as a vapour. Preferably the oxygenate and/or olefinic (co-) feed is diluted with a diluent gas. Any diluent gas known by the skilled person to be suitable for such purpose can be used. Preferably an inert gas is used such as for example argon, nitrogen and steam. For example, the feeds can be diluted with steam, for example in the range from 0.01 to 10 kg steam per kg feed.

In a further preferred embodiment small amounts of water are added in order to improve the stability of the catalyst by reducing coke formation.

The invention is illustrated by the following nonlimiting examples.

### Example 1

To simulate the start-up with an ex-situ co-feed a plug flow, isothermal reactor model is constructed from the measured alkylation and cracking kinetics of a TON-type zeolite catalyst having a silica to alumina ratio of 100 using Aspen Custom Modeler (ACM). Table 1 shows the feed and product molar flow rates for a single- pass reactor using a feed of di-methyl ether (DME) and iso-butene operated at 1 bar, 415 °C and containing 50 tonnes of the zeolite catalyst.

**Table 1: Single-pass reactor**

| Component | Feed Stream (kmol/hour) | Product Stream (kmol/hour) |
|---|---|---|
| C₂⁼ | 0 | 164 |
| C₃⁼ | 0 | 2118 |
| C₄⁼ | 1200 | 1539 |
| C₅+⁼ | 0 | 23 |
| MeOH | 0 | 1 |
| DME | 4077 | 0 |
| H₂O | 0 | 4076 |

Table 2 shows the feed, product and recycle streams for the same reactor operated with a feed of DME without a co-fed olefin but where a recycle stream is applied consisting of all C₄= and higher olefins as well as any unreacted DME and methanol that appear at the reactor outlet. The total feed consists of the feed stream and the recycle stream. The total product consists of the product stream and the recycle stream. As can be seen in table 2, further an improvement in propylene yield can be obtained.

**Table 2: Reactor with recycle**

| Component | Feed Stream (kmol/hour) | Product Stream (kmol/hour) | Recycle Stream (kmol/hour) |
|---|---|---|---|
| C₂⁼ | 0 | 227 | 0 |
| C₃⁼ | 0 | 2567 | 0 |
| C₄⁼ | 0 | 0 | 1842 |
| C₅+⁼ | 0 | 0 | 3 |
| MeOH | 0 | 0 | 0 |
| DME | 4077 | 0 | 0 |
| H₂O | 0 | 4077 | 0 |

### Comparative example A

In order to simulate the start-up of the process without an ex-situ olefinic co-feed, methanol was reacted over a MTT zeolite with a silica-to-alumina ratio of 48. The reactor was heated in argon to the reaction temperature and a mixture consisting of 8 vol.% methanol in argon was passed over the catalyst at atmospheric pressure at a flow rates of 100 ml/min. Gas hourly space velocity (GHSV) is 60,000, based on total gas flow. Weight hourly space velocities (WHSV) is 6.9 gram methanol/gram catalyst/hr, based on methanol mass flow. The effluent from the reactor was analyzed by mass spectrometry to determine the product composition. The following tables (table 3) lists reaction parameters together with the compositional data, as determined by GC:

**Table 3**

| Catalyst | MTT-type |
|---|---|
| GHSV, ml/gram/hr | 60,000 |
| Temperature °C | 500°C |
| Time on stream, hr | 1 |
| Methanol conversion, % | 75 |
| Methanol conc., vol.% | 2.0 |
| DME conc., vol.% | 3.0 |
| Total olefin conc., vol.% | < 0.2 |

As can be seen from the above a start-up or initiating step in a MTT type zeolite catalyzed process without any olefinic co-feed results in a low conversion to olefins.

It is therefore advantageous to use an ex-situ obtained olefinic co-feed to start up such a process.

### Example 2 and comparative example B

In this example dimethylether (DME) was reacted over a MFI-type zeolite with a silica-to-alumina ratio (SAR) of 55 with or without 1-butene as co-feed.

A sample of zeolite powder was pressed into tablets and the tablets were broken into pieces and sieved. For catalytic testing, the sieve fraction of 40-60 mesh has been used. Prior to reaction, the fresh catalyst in its ammonium-form was treated ex-situ in air at 600 °C for 4 hours.

The reaction was performed using a quartz reactor tube of 3.6 mm internal diameter. The catalyst was heated in argon to the reaction temperature. A feed consisting of 3 vol.% dimethylether in Argon (comparative) and a feed consisting of 3 vol% dimethylether and 3 vol% 1-butene in Argon was passed over the catalyst at atmospheric pressure (1 bar). Gas hourly space velocity is based on total gas flow (ml.g_{cat}⁻¹.h⁻¹) . Periodically, the effluent from the reactor was analyzed by gas chromatography (GC) to determine the product composition. The composition has been calculated on a weight basis of all hydrocarbons analyzed. The selectivity has been defined by the division of the mass of product i by the sum of the masses of all products. The following table (table 4) lists reaction parameters together with the compositional data, as determined by GC:

**Table 4:**

| Catalyst (SAR) | MFI (55) | | | | | | | |
|---|---|---|---|---|---|---|---|---|
| Feed | 3 vol% DME (comparative) | | | | 3 vol% 1-butene ; 3 vol% DME | | | |
| Temperature (°C) | 525 | | | | 525 | | | |
| GHSV (ml.g_{cat}⁻¹.h⁻¹) | 15,000 | | 60,000 | | 15,000 | | 60,000 | |
| Time on stream (h) | 0 | 2.2 | 0 | 2.2 | 0 | 2.2 | 0 | 2..2 |
| Methane (wt%) / | 2.1 / | 7.4 / | 1.9 / | 9.1 / | 0.96 / | 1.4 / | 0.47 / | 1.1 / |
| Selectivity (%) | 2.2 | 7.4 | 1.9 | 9.0 | 1.1 | 1.6 | 0.6 | 1.3 |
| Ethylene (wt%) / | 25.3 / | 21.8 / | 18.9 / | 16.5 / | 25.9 / | 24.4 / | 19.8 / | 15.7 / |
| Selectivity (%) | 29.4 | 24.9 | 21.6 | 18.6 | 35 | 32.5 | 27.2 | 21.8 |
| Propylene (wt%) / | 33.9 / | 29.9 / | 38.9 / | 31.3 / | 30.6 / | 30.3 / | 37.2 / | 36 / |
| Selectivity (%) | 39.4 | 34.3 | 44.3 | 35.3 | 41.3 | 40.3 | 51.1 | 50.1 |
| Butene isomers (wt%) / | 8.7 / | 7.4 / | 11.4 / | 9.2 / | 9.8 / | 10.1 / | 14.5 / | 15.2 / |
| Selectivity (%) | 10.1 | 8.5 | 13.0 | 10.3 | -* | -* | -* | -* |
| Pentene isomers (wt%) / | 2.7 / | 2.6 / | 4.1 / | 3.3 / | 3.0 / | 4.4 / | 4.7 / | 6.5 / |
| Selectivity (%) | 3.2 | 3.0 | 4.6 | 3.7 | 4.1 | 5.9 | 6.4 | 9.0 |
| Hexene isomers (wt%) / | 2.3 / | 3.1 / | 2.9 / | 3.6 / | 2.8 / | 3.5 / | 2.9 / | 4.1 / |
| Selectivity (%) | 2.7 | 3.5 | 3.3 | 4.0 | 3.8 | 4.6 | 4.0 | 5.7 |
| C₇-C₉ (wt%) / | 11.2 / | 16.1 / | 9.9 / | 16.9 / | 10.9 / | 11.3 / | 7.8 / | 8.7 / |
| Selectivity (%) | 13.0 | 18.4 | 11.3 | 19.1 | 14.7 | 15.1 | 10.7 | 12.1 |

| | | | | | | | | |
|---|---|---|---|---|---|---|---|---|
| * C₄⁼ isomers were excluded from selectivity calculations, since C₄⁼ was used as co-feed in these experiments. | | | | | | | | |

As can be seen from the above date in table 4, catalyst degradation and methane make are substantially less if the process is started up with an ex-situ provided olefinic co-feed.

## Claims

1. Process for the preparation of an olefin comprising:
a) reacting an oxygenate feed and an olefinic co-feed, in a ratio of mol oxygenate to mol olefin in the range of 10:1 to 1:10, in a reactor in the presence of a one-dimensional zeolite having 10-membered ring channels to obtain an reaction product containing one or more olefins;
b) separating the olefinic reaction product obtained in step a) in at least a product fraction containing olefins and a further fraction containing olefins;
c) recycling at least part of the further fraction containing olefins to step a);
wherein the olefinic co-feed in step a) is at least partially obtained ex-situ during a first phase and wherein the olefinic co-feed in step a) consists essentially of recycled olefins from step c) during a subsequent second phase.

2. Process according to claim 1, wherein the olefinic co-feed consists of olefins having 4 or more carbon atoms.

3. Process according to claim 1, wherein the olefinic co-feed consists of olefins having 4 or less carbon atoms.

4. Process according to claim 1 wherein the olefinic co-feed contains ethene, propene and/or butenes, which are obtained by cracking heptenes and/or octenes derived from the reaction product.

5. Process according to claim 1 wherein the olefinic co-feed contains butenes, pentenes and/or hexenes, which are obtained by oligomerization of ethene and/or propene, derived from the reaction product.

6. Process according to claim 1, wherein the part of the olefinic co-feed obtained ex-situ gradually decreases in time and the part of the olefinic co-feed obtained in-situ gradually increases in time.

7. Process according to any one of claims 1 to 6, wherein the oxygenate is methanol or dimethylether.

8. Process according to any one of claims 1 to 7 wherein the zeolite of the MTT- or TON-type.

9. Process according to any one of claims 1-8, wherein the ratio of mol oxygenate to mol olefin lies in the range of 5:1 to 1:5.

## Patentansprüche

1. Verfahren zur Herstellung eines Olefins, umfassend:
a) Umsetzen eines Oxygenateinsatzmaterials und eines olefinischen Co-Einsatzmaterials in einem Verhältnis von Mol Oxygenat zu Mol Olefin im Bereich von 10:1 bis 1:10 in einem Reaktor in Gegenwart eines eindimensionalen Zeoliths mit 10-gliedrigen Ringkanälen, um ein ein oder mehrere Olefine enthaltendes Reaktionsprodukt zu erhalten;
b) Auftrennen des im Schritt a) erhaltenen olefinischen Reaktionsprodukts in wenigstens eine Olefine enthaltende Produktfraktion und in eine Olefine enthaltende weitere Fraktion;
c) Recyclieren von wenigstens einem Teil der Olefine enthaltenden weiteren Fraktion zu Schritt a);
wobei das olefinische Co-Einsatzmaterial im Schritt a) während einer ersten Phase wenigstens teilweise ex-situ erhalten wird und wobei das olefinische Co-Einsatzmaterial im Schritt a) während einer darauf folgenden zweiten Phase im Wesentlichen aus recyclierten Olefinen aus dem Schritt c) besteht.

2. Verfahren nach Anspruch 1, wobei das olefinische Co-Einsatzmaterial aus Olefinen mit 4 oder mehr Kohlenstoffatomen besteht.

3. Verfahren nach Anspruch 1, wobei das olefinische Co-Einsatzmaterial aus Olefinen mit 4 oder weniger Kohlenstoffatomen besteht.

4. Verfahren nach Anspruch 1, wobei das olefinische Co-Einsatzmaterial Ethen, Propen und/oder Butene enthält, welche durch Cracken von Heptenen und/oder Oktenen, die aus dem Reaktionsprodukt herrühren, erhalten werden.

5. Verfahren nach Anspruch 1, wobei das olefinische Co-Einsatzmaterial Butene, Pentene und/oder Hexene enthält, welche durch Oligomerisieren aus Ethen und/oder Propen, die aus dem Reaktionsprodukt herrühren, erhalten werden.

6. Verfahren nach Anspruch 1, wobei sich der Teil des ex-situ erhaltenen olefinischen Co-Einsatzmaterials mit der Zeit allmählich verringert und sich der Teil des in-situ erhaltenen olefinischen Co-Einsatzmaterials mit der Zeit allmählich erhöht.

7. Verfahren nach einem der Ansprüche 1 bis 6, wobei das Oxygenat Methanol oder Dimethylether ist.

8. Verfahren nach einem der Ansprüche 1 bis 7, wobei der Zeolith vom MTT- oder vom TON-Typ ist.

9. Verfahren nach einem der Ansprüche 1 bis 8, wobei das Verhältnis von Mol Oxygenat zu Mol Olefin im Bereich von 5:1 bis 1:5 liegt.

## Revendications

1. Procédé pour la préparation d'une oléfine comprenant les étapes consistant à :
a) faire réagir une charge d'oxygénat et une co-charge oléfinique, dans un rapport de nombre de moles d'oxygénat au nombre de moles d'oléfine situé dans la plage de 10:1 à 1:10, dans un réacteur en présence d'une zéolite unidimensionnelle présentant des canaux formés de cycles à 10 éléments, pour obtenir un produit réactionnel contenant une ou plusieurs oléfines ;
b) séparer le produit réactionnel oléfinique obtenu à l'étape a) dans au moins une fraction de produit contenant des oléfines et une autre fraction contenant des oléfines ;
c) recycler au moins une partie de l'autre fraction contenant des oléfines à l'étape a) ;
dans lequel la co-charge oléfinique à l'étape a) est au moins en partie obtenue de manière ex situ au cours d'une première phase et dans lequel la co-charge oléfinique à l'étape a) est constituée essentiellement d'oléfines recyclées provenant de l'étape c) au cours d'une seconde phase successive.

2. Procédé selon la revendication 1, dans lequel la co-charge oléfinique est constitué d'oléfines ayant 4 atomes de carbone ou plus.

3. Procédé selon la revendication 1, dans lequel la co-charge oléfinique est constitué d'oléfines ayant 4 atomes de carbone ou moins.

4. Procédé selon la revendication 1, dans lequel la co-charge oléfinique contient de l'éthène, du propène et/ou des butènes, qui sont obtenus par craquage d'heptènes et/ou d'octènes dérivés du produit réactionnel.

5. Procédé selon la revendication 1, dans lequel la co-charge oléfinique contient des butènes, des pentènes et/ou des hexènes, qui sont obtenus par oligomérisation de l'éthène et/ou du propène, dérivés du produit réactionnel.

6. Procédé selon la revendication 1, dans lequel la partie de co-charge oléfinique obtenue ex situ diminue de manière graduelle dans le temps et la partie de co-charge oléfinique obtenue in situ augmente de manière graduelle dans le temps.

7. Procédé selon l'une quelconque des revendications 1 à 6, dans lequel l'oxygénat est le méthanol ou l'éther de diméthyle.

8. Procédé selon l'une quelconque des revendications 1 à 7, dans lequel la zéolite est du type MTT ou TON.

9. Procédé selon l'une quelconque des revendications 1 à 8, dans lequel le rapport du nombre de moles d'oxygénat au nombre de moles d'oléfine se situe dans la plage de 5:1 à 1:5.
